# EUROPEAN PATENT SPECIFICATION

(11) **EP 2 123 249 B1**
(45) Date of publication and mention of the grant of the patent: **22.07.2020**
(21) Application number: 08710994.8
(22) Date of filing: 08.02.2008
(51) Int. Cl.: A61K 8/39, A61Q 19/00, C10M 105/36, C10N 30/00, C10N 40/00

(54) **OIL SOLUTION, AND LUBRICANT, MOISTURE-RETAINING AGENT AND PREPARATION FOR EXTERNAL APPLICATION CONTAINING THE SAME**
ÖLLÖSUNG UND GLEITMITTEL, FEUCHTIGKEIT ZURÜCKHALTENDES MITTEL UND DIESE ENTHALTENDE ZUBEREITUNG ZUR TOPISCHEN APPLIKATION
SOLUTION HUILEUSE ET AGENT LUBRIFIANT PRÉSERVANT L'HYDRATATION ET PRÉPARATION POUR APPLICATION EXTERNE CONTENANT CES SUBSTANCES

(30) Priority: 09.02.2007 JP 2007030323
(43) Date of publication of application: 25.11.2009
(73) Proprietor: Kokyu Alcohol Kogyo Co., Ltd., Narita-shi Chiba 287-0225 (JP)
(72) Inventor: Takeda, Kyoichi, Narita-shi Chiba 287-0225 (JP); Kokeguchi, Yuki, Narita-shi Chiba 287-0225 (JP); Kawai, Kiyotaka, Narita-shi Chiba 287-0225 (JP)
(74) Representative: Nuss, Laurent
(86) International application number: PCT/JP2008/052115
(87) International publication number: WO 2008/096845

(56) References cited:
- JP-A- 06 184 488
- JP-A- 2001 335 465
- JP-A- 2001 342 116
- JP-A- 2001 342 118
- JP-A- 2002 047 122
- JP-A- 2002 047 164
- JP-A- 2002 053 450
- JP-A- 2002 114 624
- JP-A- 2006 176 748
- US-A- 3 623 987
- US-A- 3 635 853
- US-A1- 2003 091 603

## Description

### [Technical field]

The present invention relatesto an oil agent having superior water solubility and oil solubility as well as suppressed stickiness when applied to skin, and to a lubricating agent, a moisturizing agent and an external preparation containing said oil agent.

### [Background art]

While there is a large demand for water-soluble oil agents, conventionally-known water-soluble or water-containing oil agents have a high viscosity so that they have some problems in the feeling of use when applied to skin. For example, glycerin is used in moisturizing agents, etc. to prevent the evaporation of water from the skin by forming a film on the skin surface; however, while it is necessary to increase the amount of blending the glycerin in order to obtain moist feeling when applied to the skin, when the amount of glycerin blending is sufficiently increased to obtain moist feeling, the feeling of oiliness also increases to generate stickiness, and therefore good feeling of use cannot be obtained.

Under such circumstance, with the aim of increasing moisturizing effects and suppressing stickiness, an external preparation such as a cosmetic material which contains an oligomer ester synthesized from divalent carboxylic acid and polyglycerin (Journal of Technical Disclosure of Japan Institute of Invention and Innovation (JIII) No. 2006-501820, Journal of Technical Disclosure of Japan Institute of Invention and Innovation (JIII) No. 2006-504729), and a lubricating agent having water solubility, lubrication and anti-rusting characteristics which contains the above oligomer ester (Journal of Technical Disclosure of Japan Institute of Invention and Innovation (JIII) No. 2006-505668) have been proposed. However, because these oligomer esters have high molecular weights and high viscosities, they can provide moisturizing effects but cannot sufficiently prevent stickiness when used as a cosmetic material.

A shampoo containing a plant-oil-derived water-soluble ester, a cation polymer, a pseudo-kaolinic polyamine, and a surfactant has been proposed with the aim of increasing the adsorption of treatment components that are applied to the hair after shampooing (JP A No. 2006-347972). Here, as the plant-oil-derived water-soluble ester, polyethylene glycol esters of plant oils such as jojoba oil, sunflower oil, macadamia ternifolia seed oil, olive oil, almond oil and castor oil have been listed; however, such esters derived from plant oils show stickiness when applied to skin, and therefore satisfactory good feeling of use cannot be obtained. JP 2001-342116 A discloses a hair cosmetic in form of a hair conditioning agent. It uses an oil component dissolved in water in an amount of more than 15 % by weight but then the lipophilicity is poor and the feel with use, and in particular, the smoothness is not felt.

Furthermore, in general, water-soluble oil agents are hardly soluble in oil, and oil-soluble oil agents are hardly soluble in water. Oil agents that satisfy both of these features are not known. Therefore, in order to achieve a wide range of application of oil agents, an oil agent which is soluble in both water and oil, and shows no stickiness when applied to skin, has been strongly desired.

### [Disclosure of the invention]

### [Problem to be solved by the invention]

Therefore, an object of the present invention is to provide an oil agent having solubility in water and in oil which offers excellent feeling of use without stickiness when applied to skin, as well as a lubricating agent, moisturizing agent and external preparation containing said oil agent.

### [Means of solving the problem]

As a result of an intensive investigation taking into account the above-mentioned object, the present inventors have found that an ester consisting of a specific dibasic acid and a poly- (or mono-) ethylene glycol monoether has excellent solubility in water and in oil, and offers excellent feeling of use without stickiness when applied to skin, and the present invention has thus been accomplished.

Namely, the present invention relates the use of an oil agent in the form of dicarbitol succinate, said oil agent having a viscosity of 5 - 200 mPa.s at 25°C (measured using a Brookfield viscometer DV-II+ Spindle No. 2, 12 rpm) for the manufacturing of a preparation for external use on skin, hair, mucosa and wounds. Other advantageous variants of uses are given in the dependent claims.

Furthermore, the present invention relates to said oil agent, wherein the solubility in water is 5 % by mass or more at 20°C.

In addition, the present invention relates to said oil agent, wherein the viscosity is 5-200 (mPa·s) at 25°C.

Furthermore, the present invention relates to an external preparation comprising said oil agent.

In addition, the present invention relates to said external preparation, wherein it is a cosmetic material.

The oil agent of the present invention contains an ester compound of general formula I which consists of a specific dibasic acid and a specific polyethylene glycol monoether, as mentioned above. Such an ester compound has a low viscosity and good solubility in water; therefore, by blending the oil agent into moisturizing lotions (cosmetic solution), hair-care products (in particular shampoos and hair mists, etc.) and make-up cosmetic materials (lip-care products and foundations, etc.), moisturizing agents and external preparations including cosmetic materials which offer appropriate tightness and moist feeling to the skin without stickiness can be provided. In addition, because the inventive oil agent has good solubility in water and in oil, it has a wide range of applicability, so that it can be applied to various fields such as in lubricating agents, paints and inks, etc.

### [Brief description of the Drawings]

[fig.1] Diagram showing an IR chart of dicarbitol succinate.
[fig.2A] Diagram showing a gas chromatography data from a GC/MS chart of dicarbitol succinate.
[fig.2B] Diagram showing a mass spectrometry data from a GC/MS chart of dicarbitol succinate.

### [Best mode for carrying out the invention]

### [1] Oil agent

The oil agent of the present invention is dicarbitol succinate.

The inventive oil agent has a high solubility in water. The water solubility of the inventive oil agent at 20°C is preferably 5 % by mass or more, more preferably 10 % by mass or more, and furthermore preferably the oil agent can be dissolved in water at any ratio.

The inventive oil agent also has a high solubility in oil in addition to water. For example, the solubility in KAK99 (isononyl isononanoate) of the inventive oil agent at 20°C is preferably 5 % by mass or more, more preferably 10 % by mass or more, and furthermore preferably the oil agent can be dissolved in KAK99 at any ratio.

The viscosity of the oil agent of the present invention is, when used in external preparations such as moisturizers and cosmetic materials, preferably 5-200 (mPa·s, 25°C), and more preferably 8-100 (mPa·s, 25°C) . When the viscosity is within the above range, good feeling of use without stickiness can be obtained upon application to skin, etc. In addition, when the oil agent is used in lubricating agents, the viscosity is preferably 50-200 (mPa·s, 25°C), and more preferably 60-180 (mPa·s, 25°C). When the viscosity is within this range, appropriate lubricating property and workability can be obtained. In this specification, "viscosity" refers to a value of viscosity measured using a Brookfield viscometer DV-II+ (Spindle No. 2, 12 rpm, 25°C).

The acid value of the oil agent of the present invention is preferably 3 or less, and more preferably 1 or less. When an oil agent has a high acid value, it causes odor and irritation in the skin; accordingly, a low acid value is preferred. The hydroxyl value of the oil agent is preferably 300 or less, and more preferably 150 or less. When a hydroxyl value is within this range, the viscosity becomes low, leading to refreshing feeling of use and water solubility of the oil agent.

The color (APHA) is desirably low for external preparations such as moisturizers and cosmetic materials, and is preferably 50 or less, and more preferably 20 or less.

### [2] Process for producing oil agent

A process for producing the oil agent of the present invention is not particularly limited, and may employ a well-known method, or an appropriate combination of well-known methods. In general, the inventive oil agent can be synthesized by the ester condensation of a dibasic acid and a poly- (or mono-)ethylene glycol monoether. The reaction temperature of the ester condensation is preferably 120-160°C. Examples of a catalyst include sodium hydroxide, p-toluenesulfonic acid, boron trifluoride, hydrogen fluoride, tin chloride, zinc, titanium, potassium hydroxide, mineral acid (sulfuric acid, hydrochloric acid, etc.), zinc chloride, hypophosphorous acid, and dibutyltin oxide etc., and it is preferable to use p-toluenesulfonic acid, hypophosphorous acid, etc. The reaction may also be carried out without a catalyst. Examples of a reaction solvent include benzene, toluene and xylene, etc., and it is preferable to use toluene, xylene, etc. The reaction may also be carried out without a solvent. The ester condensation reaction is carried out, for example, using an acid value as the indicator, and it is carried out until the acid value becomes preferably 3 or less, and more preferably 1 or less.

### [3] External preparation

The external preparation of the present invention is limited as long as it is an external preparation comprising the inventive oil agent and is applicable to skin, hair, mucosa, wounds, etc. ; it includes various types of cosmetic materials, medicinal drugs, etc.

Examples of the cosmetic materials include emulsions, creams (skin cream, lip cream, hair cream, etc.), liquid foundation, eyeliner, mascara, eye shadow gel, lipstick, lipgloss, eye gloss, eye color, blusher, body gloss, ointments, soaps, mousses, tonics, gels, shampoo, hair mist, bath agents, and nail care products.

Examples of the medicinal drugs and quasi drugs include preparations such as lotions, creams, ointments, sprays, aerosols, skin patches, and gels. Examples of active medicinal agents include anti-infective agents (antiviral agents, etc.), analgesics or analgesic mixtures, arthritis drugs, antidepressants, diabetes drugs, antihistamine agents, anti-inflammatory agents, migraine preparations, antiemetic agents, antitumor agents, antipruritics, psychosis drugs, xanthine derivatives, calcium channel blockers, beta blockers, antiarrhythmic agents, antihypertensive agents, diuretics, cardiovascular preparations, hormones, immunosuppressive drugs, muscle relaxants, vasoconstrictors, vasodilators, wound healing promoters, allergy inhibitors, anti-acne agents, antiaging agents, antitussive agents, hemorrhoid drugs, local anesthetics, inflammation inhibitors, and anticholinergic agents.

The external preparation of the present invention may contain, depending on the intended purpose or as necessary, a skin-whitening agent, a moisturizer, an antioxidant, an anti-inflammatory agent, vitamins, a hormonal agent, an enzyme, a circulation promotion agent, aminoacids, anUV-absorbing agent, a sunscreen agent, a suntan agent, a hair growth agent (a hair-loss prevention agent, a hair growth promotion agent, etc.), an animal or plant extract, an anti-wrinkle agent, an antiseptics, a hair softener, a hair moisturizer, a makeup preparation, a hair conditioner, a skin conditioner, a hair whitening agent, a chelating agent, a cell replacement promotion agent, a coloring agent, a skin softening agent or a skin moisturizer, a deodorant or an antiperspirant, etc.

Examples of the skin-whitening agent include hydroquinone derivatives [hydroquinone glycosides such as hydroquinone α-D-glucose, hydroquinone β-D-glucose (arbutin), hydroquinone α-L-glucose, hydroquinone β-L-glucose, hydroquinone α-D-galactose, hydroquinone β-D-galactose, hydroquinone α-L-galactose, and hydroquinone β-L-galactose, etc.], kojicacid and derivatives thereof, L-ascorbic acid and derivatives thereof [L-ascorbic acidmonoesters such as L-ascorbic acid monophosphate and L-ascorbic acid 2-sulfate, L-ascorbic acid glucosides such as L-ascorbic acid 2-glucoside, and salts thereof], tranexamic acid or derivatives thereof [tranexamic acid dimer (trans-4-(trans-aminomethylcyclohexanecarbonyl)aminomethylcy clohexanecarboxylic acid hydrochloride, etc.), esters of tranexamic acid and hydroquinone (4'-hydroxyphenyl trans-4-aminomethylcyclohexanecarboxylate, etc.), esters of tranexamic acid and gentisic acid (2-(trans-4-aminomethylcyclohexylcarbonyloxy)-5-hydroxybenzo ic acid and salts thereof, etc.), amides of tranexamic acid (trans-4-aminomethylcyclohexanecarboxylic acid methylamide and salts thereof, etc.)], ellagic acid and derivatives thereof, salicylic acid and derivatives thereof [3-methoxysalicylic acid and salts thereof, 4-methoxysalicylic acid and salts thereof, 5-methoxysalicylic acid and salts thereof, etc.], resorcinol derivatives [alkylresorcinols such as 4-n-butylresorcinol and salts thereof, etc.], and plant extracts having a skin-whitening action.

Examples of the anti-inflammatory agent include glycyrrhizic acid salts (dipotassium glycyrrhizinate, ammonium glycyrrhizinate, etc.), allantoin, and mixtures thereof.

Examples of the antimicrobial agent include resorcin, sulfur, salicylic acid, zincpyrithione, photosensitizerNo. 101, photosensitizer No. 102, Octopirox, hinokitiol, bacitracin, erythromycin, neomycin, tetracycline, chlorotetracycline, benzethonium chloride, phenol, multivalent alcohols (1,2-pentanediol, 1,2-hexanediol, 1,2-octanediol, etc.) and mixtures thereof.

Examples of the vitamins include vitamins A, C, D, E, and K, vitamin A palmitate, thiamine, vitamin B₆, vitamin B₆ derivatives such as vitamin B₆hydrochloride, vitamin B₂, vitamin B₁₂, nicotinic acid, nicotinic acid derivatives such as nicotinic-acid amide, pantothenic acid, pantothenyl ethyl ether, pyridoxine, inositol, vitamin B complex such as carnitine, panthenol, and mixtures thereof.

Examples of the hormonal agent include oxytocin, corticotropin, vasopressin, secretin, gastrin, and calcitonin.

Examples of the enzyme include trypsin, lysozyme chloride, chymotrypsin, chymotrypsin-like enzymes, aspartic proteinase, semialkaliproteinase, serrapeptase, lipase, and hyaluronidase.

Examples of the antioxidant include thiotaurine, glutathione, catechin, albumin, ferritin, metallothionein, and the above-mentioned L-ascorbic acid and derivatives thereof.

Examples of blood circulation promoters include acetylcholine derivatives, cepharanthin, and carpronium chloride.

Examples of the amino acids include amphoteric amino acids such as stearyl acetyl glutamate, capryloyl silk amino acid, capryloyl collagen amino acid, capryloyl keratin amino acid, capryloyl p amino acid, cocodimonium hydroxypropyl silk amino acid, corn gluten amino acid, cysteine, glutamic acid, glycine, hair amino acids such as hair keratin amino acid and aspartic acid, threonine, serine, glutamic acid, proline, glycine, alanine, half-cystine, valine, methionine, isoleucine, leucine, tyrosine, phenylalanine, cysteic acid, histidine, arginine, cysteine, tryptophan, citrulline, lysine, silk amino acids, wheat amino acids, and mixtures thereof.

Examples of the UV-absorbing agent include benzophenone, bornelone, butyl PABA, cinnamidopropyl trimethyl ammonium chloride, disodium distyrylbiphenyl disulfonate, PABA, potassium methoxycinnamate, and mixtures thereof.

Examples of the sunscreen agent include butylmethoxydibenzoylmethane, octylmethoxycinnamate, octocrylene, octylsalicylate, phenylbenzimidazole sulfonic acid, ethyl hydroxypropylaminobenzoate, menthyl anthranilate, aminobenzoic acid, cinoxate, diethanolamine methoxycinnamate, glyceryl aminobenzoate, titanium dioxide, zinc dioxide, oxybenzone, Padimate-O, red petrolatum, and mixtures thereof.

Examples of the hair growth agent include blood circulation promoters such as swertia japonica extract, acetylcoline derivatives, cepharanthin and carpronium chloride, local stimulants such as red pepper tincture, cantharis extract and nonylic acid vanillamide, antiseborrhea agents such as pyridoxine and derivatives thereof, antimicrobial agents such as benzalkonium chloride, isopropylmethylphenol, zinc pyrithione, photosensitizer No. 101, photosensitizer No. 102, Octopirox and hinokitiol, metabolic stimulants such as photosensitizer No.301, placenta extract and biotin, amino acids such as serine, methionine and tryptophan, and vitamins such as vitamins B₂ and B₁₂, pantothenic acid and derivatives thereof.

Among the animal and plant extracts, examples of the plant extract include tea extract, rosa roxburghii extract, scutellaria root extract, houttuynia cordata extract, phellodendri cortex extract, melilotus officinalis extract, lamium album extract, glycyrrhiza extract, paeoniae radix extract, saponaria officinalis extract, luffa cylindrica extract, cinchona extract, saxifrage extract, sophora angustifolia extract, nuphar haponicum root extract, fennel extract, primrose extract, rose extract, rehmannia extract, lemon extract, lithospermum root extract, aloe extract, iris root extract, eucalyptus extract, equisetum arvense extract, sage extract, thyme extract, seaweed extract, cucumber extract, clove extract, raspberry extract, melissa officinalis extract, carrot extract, horse chestnut extract, peach extract, peach leaf extract, mulberry extract, centaurea cyanus extract, witch hazel extract, ginkgo extract, wintergreen extract, swertia japonica extract, red pepper tincture extract, and cantharis extract. Examples of the animal extract include placenta extract and collagen.

The external preparation of the present invention may contain an oily component other than the oil agent of the present invention. As the other oily component, a component of any origin such as an animal oil, a plant oil, a synthetic oil, etc. and a component with any properties such as a solid oil, a semi-solid oil, a liquid oil, a volatile oil, etc. may be used, and examples thereof include hydrocarbons, silicone oils, fats, waxes, hydrogenated oil, ester oils, fatty acids, fatty alcohols, fluorine-containing oils, and lanolin derivatives. Specific examples thereof include hydrocarbons such as light liquid isoparaffin, liquid paraffin, squalane, vaseline, polyisobutylene and polybutene, oils such as olive oil, castor oil, jojoba oil, mink oil and macadamia ternifolia seed oil, petroleum waxes such as paraffin wax and microcrystalline wax, mineral waxes such as ozokerite and ceresine, natural waxes such as carnauba wax and candelilla wax, esters such as cetyl octanoate, isopropyl myristate, isopropyl palmitate, octyldodecyl myristate, glyceryl trioctanoate, polyglyceryl diisostearate, diglyceryl triisostearate, glyceryl tribehenate, pentaerythritol rosinate, neopentyl glycol dioctanoate and cholesterol fatty acid esters, fatty acids such as stearic acid, lauric acid, myristic acid, behenic acid, isostearic acid, oleic acid and 12-hydroxystearic acid, fatty alcohols such as stearyl alcohol, cetylalcohol,laurylalcohol, oleylalcohol, isostearyl alcohol and behenyl alcohol, silicones such as low-degree-of-polymerization dimethylpolysiloxane, decamethylcyclopentasiloxane, octamethylcyclosiloxane, high-degree-of-polymerization dimethylpolysiloxane, methylphenylpolysiloxane, polyether-modified polysiloxane, a polyoxyalkylene / alkylmethylpolysiloxane / methylpolysiloxane copolymer, alkoxy-modified polysiloxane and fluorine-modified polysiloxane, fluorine-containing oils such as perfluorodecane, perfluorooctane and perfluoropolyether, and lanolin derivatives such as lanolin, lanolin acetate, lanolin fatty acid isopropyl ester and lanolin alcohol.

The external preparation of the present invention may contain, in addition to the oil agent and the above-mentioned components, as appropriate and as necessary a component that is normally used in an external preparation, such as a moisturizer, a surfactant, a UV-absorbing agent, a UV-scattering agent, a fragrance, a viscosity increasing agent, an antiseptic, acoloring agent such as an extender pigment and a coloring pigment, a pH adjusting agent, and a chelating agent (citric acid, phosphoric acid, EDTA-4Na, etc.) in a range that does not impair the effects of the present invention.

The form of the external preparation of the present invention is not particularly limited; the external preparation may be in the form of a liquid, an emulsion, a semi-solid agent, a solid agent, etc. according to its intended purpose. The emulsion is not particularly limited and may be any of a water-in-oil type (W/O type) or an oil-in-water type (O/W type), a W/O/W type, and an O/W/O type, etc. For example, in the case of a water-in-oil type emulsion cosmetic material, it is prepared by adding water and a water-soluble component (aqueous phase component) to the oil agent. The content ratio of the oil agent and the aqueous phase component may be adjusted in accordance with desired properties of the emulsion, and is not particularly limited.

### [Examples]

The present invention is explained in further detail by reference to Examples below, but the present invention should not be construed as being limited thereto.

In these examples, viscosity was measured using a Brookfield viscometer DV-II+ (Spindle No. 2, 12 rpm, 25°C).

Acid values and hydroxyl values were measured in accordance with General Test Methods, 18: Acid Value Measurement Method and 24: Hydroxyl Value Measurement Method, Japanese Standards of Cosmetic Ingredients (newly revised, first edition, 30 August 1999). In addition, color (APHA) was measured using OME2000 (Nippon Denshoku Industries, Co., Ltd.).

### Example 1

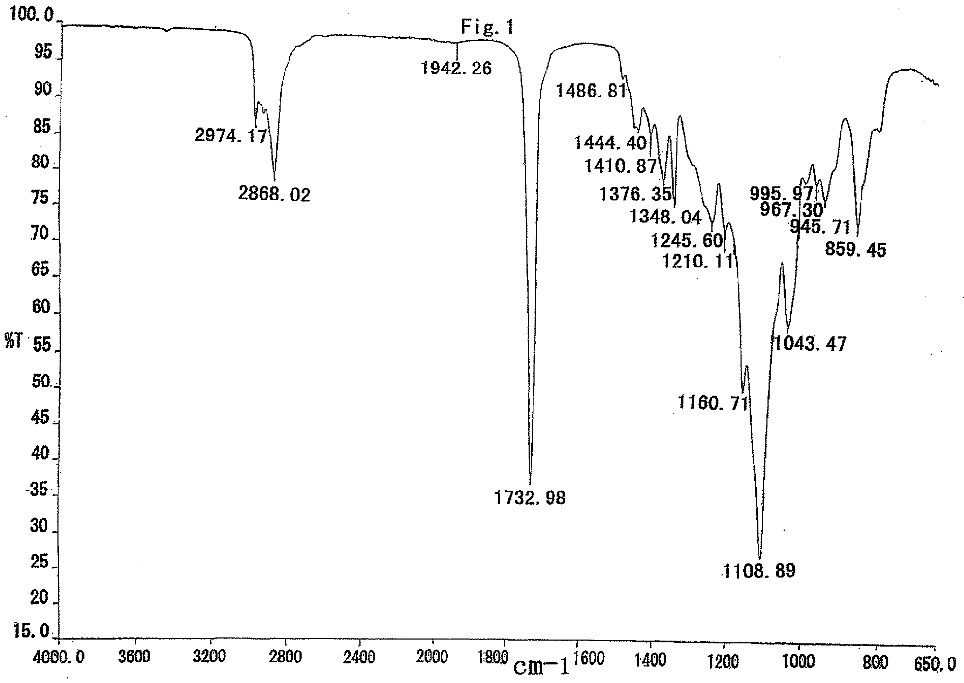

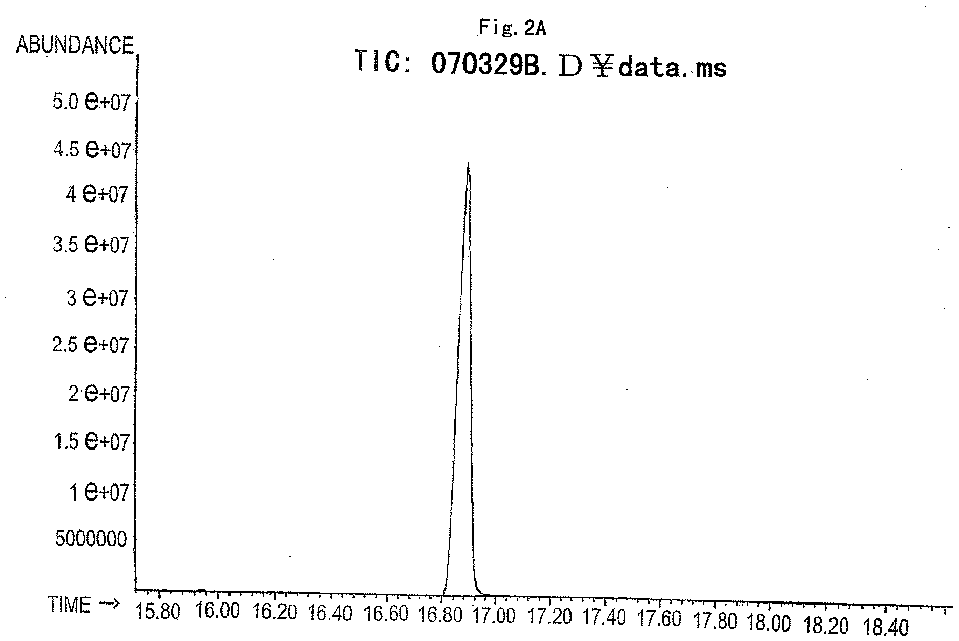

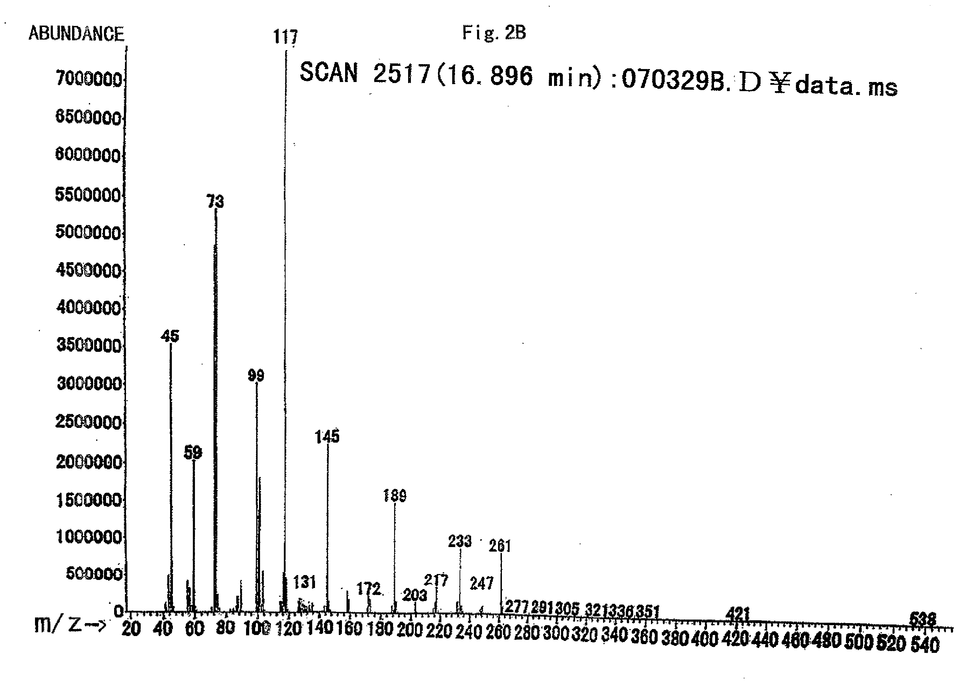

To a four-necked 1-L flask equipped with a stirring device, thermometer, reflux apparatus and nitrogen-gas supply nozzle, 177.2 g (1.5 mol) of succinic acid, 603.9 g (4.5 mol) of 2-(2-ethoxyethoxy)ethanol commonly known as carbitol (EDG; Yamaichi Chemical Industries, Co., Ltd.), 0.78 g of p-toluenesulfonic acid as a catalyst, and 120 ml of toluene as a solvent were added, and the reaction was carried out at 160°C under reflux until the acid value became 3 or less (OH group/COOH group = 1.5). The reaction time was approximately 15 h. Then, the reaction solution was decompressed to approximately 5 mmHg at 160°C, and unreacted carbitol was distilled away. To the reaction solution from which EDG was removed, 15.6 g of sodium carbonate decahydrate was added and stirred for 1 h at 120°C. This slurry reactant was filtered to obtain 497 g of pale yellow dicarbitolsuccinate. Figures 1 and 2A/2B show IR and GC/MS charts of the obtained dicarbitol succinate. Table 1 shows measured results of the acid value, saponification value, hydroxyl value, color (APHA), viscosity, water solubility, oil solubility and feeling of use of the dicarbitol succinate.

**[Table 1]**

| | Example 1 | Comparative example 1 |
|---|---|---|
| Acid value | 0.2 | 0.6 |
| Saponification value | 304 | 194 |
| Hydroxyl value | 0.9 | 1.5 |
| Color (APHA) | 30 | 65 |
| Viscosity ^{*1} | 12.5 | 5 |
| Water solubili-ty^{*2} | ⊚ | × |
| Oil solubili-ty^{*3} | ⊚ | ⊚ |
| Feeling of use^{*4} | light | light |

| | | |
|---|---|---|
| *1: mPa·s, 25°C *2: Evaluation: ⊚: Dissolved in water at an arbitrary ratio, ○ : Dissolved in water up to 10 % by mass, Δ: Dissolved in water up to 5 % by mass, ×: Insoluble in water. *3: Evaluation: ⊚: Dissolved in KAK99 at an arbitrary ratio, ○ : Dissolved in KAK99 up to 10 % by mass, Δ: Dissolved in KAK99 up to 5 % by mass, ×: Insoluble in KAK99. *4: Light: viscosity 5-20 (mPa·s, 25°C) Slightly light: viscosity 21-50 (mPa·s, 25°C) Moderate: viscosity 51-150 (mPa·s, 25°C) Slightly heavy: viscosity 151-250 (mPa·s, 25°C) Heavy: viscosity 251 (mPa·s, 25°C) or more | | |

### Example 2

### Production of skin lotion

Components shown in Table 3 were uniformly dissolved at 60°C-70°C, then cooled to 30°C, to prepare a skin lotion.

**[Table 2]**

| Component name | | Blending quantity (% by mass) |
|---|---|---|
| 1 | Esterified compound of Example 1 | 30.00 |
| 2 | Citric acid | 0.01 |
| 3 | Sodium citrate | 0.09 |
| 4 | Mixture of phenoxyethanol, methylparaben, ethylparaben, butylparaben, propylparaben and isobutylparaben | 0.40 |
| 5 | Purified water | Remainder |

### Example 3

### Production of skin lotion

Components shown in Table 6 were uniformly dissolved at 60°C-70°C, then cooled to 30°C, to prepare a skin lotion.

**[Table 3]**

| Component name | | Blending quantity (% by mass) |
|---|---|---|
| 1 | Esterified compound of Example 1 | 15.00 |
| 2 | EDTA-4Na | 0.05 |
| 3 | Pentylene glycol | 3.00 |
| 4 | glycerin | 2.00 |
| 5 | Fragrance | Appropriate quantity |
| 6 | Purified water | Remainder |

### Example 4

### Production of skin cream (O/W type cream)

Composition A and composition B shown in Table 8 were individually and uniformly dissolved at 75°C-80°C. Then composition B was added to composition A while stirring, and the mixture was emulsified using a homo-mixer. The mixture was subsequently cooled to 30°C while stirring, to prepare an O/W type skin cream.

**[Table 4]**

| Component name | | | Blending quantity (% by mass) |
|---|---|---|---|
| A | 1 | Squalane | 10.00 |
| | 2 | Hydrogenated Rapeseed Oil Alcohol | 1.00 |
| | 3 | Behenyl alcohol | 2.00 |
| | 4 | Polyglyceryl-2 isostearate/dimer dilinoleate copolymer | 2.00 |
| | 5 | Polyglyceryl-10 stearate | 1.50 |
| | 6 | Pentylene glycol | 3.00 |
| B | 7 | Esterified compound of Example 1 | 10.00 |
| | 8 | Citric acid | 0.01 |
| | 9 | Sodium citrate | 0.09 |
| | 10 | Xanthan gum | 0.08 |
| | 11 | Ammonium acryloyldimethyltaurate/ beheneth-25 methacrylate copolymer | 0.22 |
| | 12 | Purified water | Remainder |

### Example 5

### Production of cleansing oil

Components shown in Table 10 were uniformly dissolved at 80°C, then cooled to 30°C, to prepare a cleansing oil.

**[Table 5]**

| Component name | | Blending quantity (% by mass) |
|---|---|---|
| 1 | Esterified compound of Example 1 | 30.00 |
| 2 | Isononyl isononanoate | Remainder |
| 3 | PEG-20 glyceryl diisostearate | 10.00 |
| 4 | PEG-8 diisostearate | 8.00 |
| 5 | Polyglyceryl-2 isostearate | 5.00 |
| 6 | Polyglyceryl-2 triisostearate | 5.00 |
| 7 | Tocopherol | Appropriate quantity |

### Example 6

### Hair mist

Components 1-5 and 7 shown in Table 17 were uniformly dissolved at 80°C then cooled to 50°C. Component 6 was subsequently added, and the mixture was further cooled to 30°C to prepare a hair mist.

**[Table 6]**

| Component name | | Blending quantity (% by mass) |
|---|---|---|
| 1 | Esterified compound of Example 1 | 5.00 |
| 2 | Citric acid | 0.01 |
| 3 | Sodiumcitrate | 0.09 |
| 4 | Behenyl trimethyl ammonium chloride | 0.50 |
| 5 | Pentylene glycol | 3.00 |
| 6 | Fragrance | 0.04 |
| 7 | Purified water | Remainder |

## Claims

1. Use of an oil agent in the form of dicarbitol succinate, said oil agent having a viscosity of 5 - 200 mPa.s at 25°C (measured using a Brookfield viscometer DV-II+ Spindle No. 2, 12 rpm) for the manufacturing of a preparation for external use on skin, hair, mucosa and wounds.

2. Use according to claim 1, **characterized by** the fact that the preparation is a cosmetic material.

3. Use according to claim 1 or 2, **characterized by** the fact that the preparation is a hair care product.

4. Use according to claim 3, **characterized by** the fact that the preparation is a hair mist.

5. Use according to claim 1 or 2, **characterized by** the fact that the preparation is a make-up cosmetic material.

6. Use according to claim 1, **characterized by** the fact that the preparation is a medicinal drug including a medicinal agent chosen in the group formed by: anti-infective agents, antiviral agents, antimicrobial agents, analgesics or analgesic mixtures, arthritis drugs, antidepressants, diabetes drugs, antihistamine agents, anti-inflammatory agents, migraine preparations, antiemetic agents, antitumor agents, antipruritics, psychosis drugs, xanthine derivatives, calcium channel blockers, beta blockers, antiarrhythmic agents, antihypertensive agents, diuretics, cardiovascular preparations, hormones, immunosuppressive drugs, muscle relaxants, vasoconstrictors, vasodilators, wound healing promoters, allergy inhibitors, anti-acne agents, antiaging agents, antitussive agents, hemorrhoid drugs, local anesthetics, inflammation inhibitors, and anticholinergic agents or containing an active ingredient chosen from the group consisting of: a skin-whitening agent, a moisturizer, an antioxidant, an anti-inflammatory agent, vitamins, a hormonal agent, an enzyme, a blood circulation promotion agent, amino acids, an UV -absorbing agent, a sunscreen agent, a suntan agent, a hair growth agent, a hair-loss prevention agent, a hair growth promotion agent, an animal or plant extract, an antiwrinkle agent, an antiseptics, a hair softener, a hair moisturizer, a makeup preparation, a hair conditioner, a skin conditioner, a hair whitening agent, a chelating agent, a cell replacement promotion agent, a coloring agent, a skin softening agent or a skin moisturizer, a deodorant or an antiperspirant.

## Patentansprüche

1. Verwendung einer öligen Substanz in Form von Bis-Ethoxydiglycol Succinat, wobei die vorerwähnte ölige Substanz eine Viskosität von 5-200 mPa.s bei 25 °C aufweist (gemessen mit Brookfield-Viskosimeter DV-II+ Spindel Nr. 2 bei 12 U/min), zur Herstellung einer Zubereitung zur äußeren Anwendung auf Haut, Haar, Schleimhäuten und Wunden.

2. Verwendung gemäß Anspruch 1, **dadurch gekennzeichnet, dass** es sich bei der Zubereitung um ein Kosmetikprodukt handelt.

3. Verwendung gemäß Anspruch 1 oder 2, **dadurch gekennzeichnet, dass** es sich bei der Zubereitung um eine Haarpflegemittel handelt.

4. Verwendung gemäß Anspruch 3, **dadurch gekennzeichnet, dass** es sich bei der Zubereitung um Haarspray handelt.

5. Verwendung gemäß Anspruch 1 oder 2, **dadurch gekennzeichnet, dass** es sich bei der Zubereitung um ein Schminkmittel handelt.

6. Verwendung gemäß Anspruch 1, **dadurch gekennzeichnet, dass** es sich bei der Zubereitung um ein Medikament handelt, das einen der folgenden Arzneistoffe enthält: Antiinfektiva, Virostatika, antimikrobielle Mittel, Analgetika oder Analgetika-Mischungen, Arthritismittel, Antidepressiva, Diabetes-Medikamente, Antihistaminika, Antiinflammatorika, Migränemittel, Antiemetika, Wirkstoffe gegen Tumor, Antipruritika, Psychosemittel, Xanthinderivate, Kalziumantagonisten, Betablocker, Antiarrhythmikum, Antihypertonika, Diuretika, Herz-Kreislauf-Mittel, Hormone, Immunsuppressiva, Muskelrelaxanzien, Vasodilatatoren, Vasokonstriktoren, Wundheilmittel, Allergiemittel, Aknemittel, Anti-Aging-Produkte, Hustenmittel, Hämorrhoidalpräparate, Lokalanästhetika, Entzündungshemmer und Anticholinergika, oder das einen der folgenden Wirkstoffe enthält: Hautaufheller, Feuchtigkeitsmittel, Antioxidantien, Entzündungshemmer, Vitamine, Hormonmittel, Enzyme, Blutkreislaufmittel, Aminosäuren, UV-Schutzmittel, Sonnenschutz, Bräunungsmittel, Haarwuchsmittel, Haarausfallmittel, Mittel zur Förderung des Haarwuchses, tierische oder pflanzliche Extrakte, Faltencremes, Antiseptika, Hair Softener, Feuchtigkeitsmittel für Haar, Schminke, Conditioner, Hautpflegemittel, Haaraufhellungscremes, Komplexbildner, Zellersatzmittel, Färbemittel, Hautpflegemittel, Hautfeuchtigkeitsmittel, Deodorants oder Schweißmittel.

## Revendications

1. Utilisation d'un agent huileux sous la forme de succinate de dicarbitol, ledit agent huileux présentant une viscosité allant de 5 à 200 mPa.s à 25 °C (mesurée à l'aide d'un viscosimètre de Brookfield DV-II+, broche n° 2, 12 tr/min) pour la fabrication d'une préparation pour usage externe s'appliquant sur la peau, les cheveux, les muqueuses et les plaies.

2. Utilisation selon la revendication 1, **caractérisée par le fait que** la préparation est un produit cosmétique.

3. Utilisation selon la revendication 1 ou 2, **caractérisée par le fait que** la préparation est un produit de soin capillaire.

4. Utilisation selon la revendication 3, **caractérisée par le fait que** la préparation est une brume capillaire.

5. Utilisation selon la revendication 1 ou 2, **caractérisée par le fait que** la préparation est un produit cosmétique de maquillage.

6. Utilisation selon la revendication 1, **caractérisée par le fait que** la préparation est un médicament incluant un agent médicinal choisi parmi le groupe formé par : des agents anti-infectieux, des agents antiviraux, des agents antimicrobiens, des analgésiques ou des mélanges analgésiques, des médicaments contre l'arthrose, des antidépresseurs, des médicaments contres le diabète, des agents antihistaminiques, des agents anti-inflammatoires, des préparations contre la migraine, des agents antiémétiques, des agents antitumoraux, des antiprurigineux, des médicaments contre la psychose, des dérivés de xanthine, des bloqueurs des canaux calciques, des bêta-bloquants, des agents antiarythmiques, des agents antihypertenseurs, des diurétiques, des préparations cardiovasculaires, des hormones, des médicaments immunosuppresseurs, des relaxants musculaires, des vasoconstricteurs, des vasodilatateurs, des agents facilitant la cicatrisation de plaie, des inhibiteurs d'allergie, des agents anti-acné, des agents antivieillissement, des agents antitussifs, des médicaments contre les hémorroïdes, des anesthésiques locaux, des inhibiteurs d'inflammation, et des agents anticholinergiques ou contenant un principe actif choisi parmi le groupe constitué de : un agent d'éclaircissement de la peau, un hydratant, un antioxydant, un agent anti-inflammatoire, des vitamines, un agent hormonal, une enzyme, un agent améliorant la circulation sanguine, des acides aminés, un agent absorbant les UV, un agent de protection solaire, un agent de bronzage, un agent de pousse des cheveux, un agent de prévention de la chute des cheveux, un agent favorisant la pousse des cheveux, un extrait animal ou végétal, un agent antiride, un antiseptique, un adoucissant pour cheveux, un hydratant pour cheveux, une préparation de maquillage, un revitalisant pour cheveux, un revitalisant pour la peau, un agent d'éclaircissement des cheveux, un agent de chélation, un agent favorisant le renouvellement cellulaire, un agent de coloration, un agent d'adoucissement de la peau ou un hydratant cutané, un déodorant ou antitranspirant.
